# EUROPEAN PATENT APPLICATION

(11) **EP 0 646 795 A1**
(43) Date of publication of application: **05.04.1995**
(21) Application number: 94114304.2
(22) Date of filing: 12.09.1994
(51) Int. Cl.: G01N 33/84, G01N 31/22

(54) **Reagent composition and method for determining lithium**

(30) Priority: 23.09.1993 US 125395
(71) Applicant: Bayer Corporation, Pittsburgh, PA 15219-2502 (US)
(72) Inventor: Chapoteau, Eddy, Brooklyn, New York 11238 (US); Czech, Bronislav P., Peekskill, New York 10566 (US)
(74) Representative: Burkert, Frank

(57) **Abstract**

The invention is directed to a composition and method for determining lithium in a biological speciment utilizing the compound 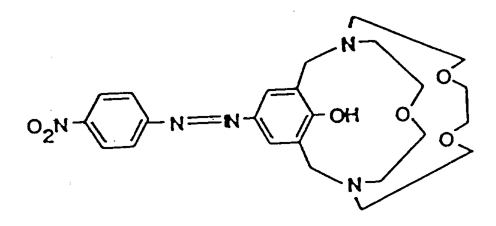
together with a buffer, a water miscible organic solvent and a nonionic surfactant. Optionally, a sodium salt is also present.

## Description

### Field Of The Invention

The present invention relates generally to the field of clinical chemistry. More particularly, the present invention relates to a lithium reagent composition and method for determining lithium present in a biological specimen.

### Background Of The Invention

The quantitative determination of lithium in clinical samples is currently typically performed by flame photometry or by ion selective electrodes (ISE). While use of a flame photometer for monitoring lithium levels is rather costly and cumbersome, the ISE method suffers mainly from interferences from sodium and other ions present in biological fluids.

A number of attempts have been made to design a colorimetric lithium assay based on lithium selective chromoionophores (Pacey et al, Synth. Commun. 11, 1981, 323-328; Kaneda et al., Tetrahedron Letters 22, 1981, 4407-4408; Ogawa et al., J. Amer. Chem. 106, 1984, 5760-5762; Sasaki et al., Anal. Chim. Acta. 174, 1985, 141-149; Misumi et al., J. Amer. Chem. Soc. 107, 1985, 4802-4803; Kimura et al., J. Org. Chem. 52, 1987, 836-844; Attiyat et al., 37, 1988, 114-121; Cram et al., J. Amer. Chem. Soc. 110, 1988, 571-577).

The various lithium selective compounds described in the prior art cited above lack adequate lithium binding in aqueous media and require extraction-photometric procedures that are difficult to automate. The relatively low level of lithium in serum (0.5-1.5 mM) imposes very high constraints on selectivity over the high normal serum sodium concentration (135-150 mM). Ideally, the selectivity for lithium over sodium should be 1,500:1 in order to essentially eliminate any sodium interference.

The first practical colorimetric lithium assay, which uses a chromogenic cryptand ionophore was described by Chapoteau et al. in Clin. Chem. 38, 1992, 1654-1657 and Czech et al. in U.S. Patent No. 5,187,103. Recently, Sutherland et al. reported in J. Chem. Soc. Chem. Commun. 1992 1716-1718 a new chromogenic cryptand for the determination of lithium in an extraction system and its potential application in optical fiber sensors.

### Summary Of The Invention

The present invention is directed toward a reagent composition and process for the colorimetric determination of lithium in aqueous clinical samples. The reagent composition is based on the chromogenic cryptand I. 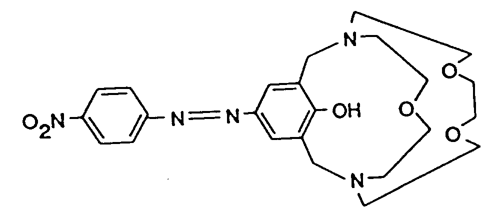
Unlike the chromogenic cryptand described by Chapoteau and Czech, chromoionophore I, as disclosed by Sutherland et al., is not totally selective for lithium over sodium.

According to the present invention a sodium salt at a concentration less than 2 x 10⁻² M; a base to maintain the pH at 12 or higher; a water-miscible organic solvent at a concentration less than 10% volume to volume; and a non-ionic surfactant at a concentration less than 10% weight to volume are added to the chromoionophore I to form the reagent composition. As an alternative to the addition of base a buffer can be added to adjust the pH to about 11. When this is done the presence of sodium salt is optional.

### Description Of The Preferred Embodiments

The following definitions are provided to clarify the scope of the present invention, and to enable formulation of the reagent composition and use thereof. As used herein, "chromogenic" is intended as meaning that characteristic of a chemical system whereby a detectable response is generated in response to an external stimulus. Thus, for example, an ionophore is chromogenic when it is capable of exhibiting a detectable response upon complexing with an ion, which detectable response is not limited solely to change in color as defined below.

The expression "detectable response" means a change in or appearance of a property in a system which is capable of being perceived, either by direct observation or instrumentally, and which is a function of the presence of a specific ion in an aqueous test sample. Some examples of detectable responses are the changes in or appearance of color, fluorescence, phosphorescence, reflectance, chemiluminescence, or infrared spectrum. These are referred to generally as chromogenic responses. Other examples of detectable responses are changes in electrochemical properties, pH and nuclear magnetic resonance.

In addition to the chromoionophore I, a sodium salt is normally required at a concentration less than 2 x 10⁻² M. The sodium salt normalizes sodium interference. Suitable sodium salts includes sodium chloride, sodium bromide, sodium acetate and sodium bicarbonate. The preferred sodium salt is sodium chloride.

The water-miscible organic solvent should be present at a concentration less than 10% volume to volume. The preferred concentration of water-miscible organic solvent is 1% volume to volume. Suitable organic solvents include cyclic ethers such as dioxane and tetrahydrofuran; ethylene glycol derivatives such as monoethyl diethylene glycol, monoethyl diethylene glycol, monopropyl diethylene glycol, monobutyl diethylene glycol; amides such as formamide, dimethylformamide, pyrrolidine, N-alkyl pyrrolidine (methyl); aliphatic alcohols such as methanol, ethanol, propanols and butanols; sulfoxides such as dimethylsulfoxide; amino alcohols such as ethanolamine, propanolamine, amino propanediols; and ketones such as acetone, and methylethylketone. A particularly preferred water-miscible organic solvent is diethylene glycol monoethyl ether (DEGMEE).

The reagent composition also includes a strong base to maintain a pH environment of at least 12. Suitable bases include potassium hydroxide, rubidium hydroxide, cesium hydroxide, tetramethylammonium hydroxide and tetraethylammonium hydroxide. A preferred base is potassium hydroxide.

The lithium assay can also be run in sodium hydroxide but the concentration of the base must be low enough (0.05-0.10 M) to allow measurement of lithium. Alternatively, the lithium assay can be performed under conditions where the pH is strictly controlled to minimize the sodium response without the addition of a sodium compound. Thus, an appropriate buffer to maintain pH between 11.1 and 11.3 is incorporated into the reagent. Suitable buffers include cyclohexylaminopropanesulfonic acid (CAPS), arginine and γ-aminobutyric acid.

The reagent composition can also include a nonionic surfactant to solubilize and stabilize the lithium complex being formed. Normally, the nonionic surfactant is present in a concentration of less than 10% on a weight to volume basis. Suitable surfactants include nonionic surfactants such as Brij-35 [polyoxyethylene (23) lauryl ether; ICI] and Triton X-100 [t-octylphenoxypolyethoxyethanol; Sigma Chemical Co.].

In addition, the reagent composition can contain manufacturing excipients and other inert ingredients, all of which are easily within the knowledge of one skilled in the art, or which could be routinely determined without the need for undue experimentation. The reagent composition can be in a liquid form when used, or can be impregnated into a suitable carrier matrix to form a test device. The test device can take on such formats as a dip-and-read strip for urine or a test slide for use with an automatic blood analyzer, or can form a multilayer structure such as described in U.S. Patent Nos. 3,992,158 and 4,292,272.

The carrier matrix is preferably filter paper. Other materials useful as an absorbent carrier include felt, porous ceramic strips, woven matted glass fibers (described in U.S. Patent No. 3,846,247). Also suggested as suitable absorbent carriers of test strips are materials such as wood, cloth, sponge materials and argillaceous substances (as described in U.S. Patent No. 3,552,928).

The new colorimetric reagent composition and method described in this application can be applied in clinical diagnostics for a rapid, easy-to-perform, accurate quantitation of lithium in biological fluids. Due to the simplicity and convenience of its use the colorimetric method is superior to the current methods, i.e., flame photometry and ion-selective electrodes. It is believed that in a short time the colorimetric lithium assay will replace the currently used methods.

It was known from the prior art (Czech et al., U.S. Patent No. 5,187,103) that the high lithium over sodium selectivity is required for a chromoionophore to be useful in the lithium assay. Since chromoionophore I, which was primarily designed for use in optical fiber sensors, binds both lithium and sodium in homogenous aqueous solutions, thus lacking adequate lithium selectivity, it was unobvious to apply it to a colorimetric reagent used to assay lithium.

### EXAMPLES

The following examples set forth various aspects of the subject invention. It will be understood that the formulations which follow are provided for the purposes of illustration only and that other ingredients, proportions and procedures can be employed in accordance with the disclosure of this invention.

### Example 1

A reagent composition was prepared by predissolving 2.5 mg of chromoionophore I with 1.0 mL (milliliter) diethylene glycol monoethyl ether (DEGMEE) and adding the solution to 100 mL of 1M TMAOH (tetramethylammonium hydroxide).

2.0 mL of the reagent composition was mixed at room temperature with 0.04 mL of sample in a cuvette which was placed in a Cary 3 spectrophotometer where absorbances were read at various wavelengths. The first sample was an aqueous lithium chloride solution and the second sample an aqueous sodium chloride solution.

| **Lithium response** | | |
|---|---|---|
| [Li⁺]x10⁻³ M | A₅₀₀ | A₆₀₀ |
| 0.0 | 0.687 | 1.420 |
| 0.5 | 0.888 | 1.077 |
| 1.0 | 1.098 | 0.735 |
| 3.0 | 1.390 | 0.201 |

| **Sodium response** | | |
|---|---|---|
| [Na⁺]x10⁻¹ M | A₅₀₀ | A₆₀₀ |
| 0.0 | 0.687 | 1.420 |
| 100 | 1.051 | 1.133 |
| 120 | 1.061 | 1.131 |
| 160 | 1.067 | 1.118 |

This example demonstrates that under the above described conditions the chromoionophore responds to both lithium and sodium and the reagent cannot be used for determining lithium in clinical samples which contain sodium.

### Example 2

A reagent composition was prepared by dissolving 2.5 mg of chromoionophore I in 1.0 mL of DEGMEE and adding the solution to 100 mL of 1 M TMAOH containing 0.23 g (grams) of sodium chloride.

2.0 mL of the reagent composition was mixed at room temperature with 0.04 mL sample in a cuvette and the absorbances were read as in Example 1.

| **Lithium response** | | |
|---|---|---|
| [Li⁺]x10⁻³ M | A₅₀₀ | A₆₀₀ |
| 0.0 | 1.144 | 1.259 |
| 0.5 | 1.218 | 0.963 |
| 1.0 | 1.294 | 0.705 |
| 3.0 | 1.487 | 0.264 |

Under the conditions of this example the reaction mixture became hazy after 2 minutes indicating the need for surfactant.

### Example 3

A reagent composition was prepared by predissolving 2.5 mg of chromoionophore I in 1.0 mL of DEGMEE and adding the solution to 100 mL of 1 M TMAOH containing 1.0 g of Triton X-100 (t-oxtylphenoxypolyethoxyethanol) and 0.23 g of sodium chloride.

2.0 mL of the reagent composition was the mixed at room temperature with 0.03 mL of sample in a cuvette and absorbances were read on a spectrophotometer as in Example 1

| **Lithium response** | | |
|---|---|---|
| [Li⁺]x10⁻³ M | A₅₀₀ | A₆₀₀ |
| 0.0 | 0.914 | 1.330 |
| 0.5 | 1.016 | 1.115 |
| 1.0 | 1.096 | 0.888 |
| 2.0 | 1.282 | 0.475 |
| 3.0 | 1.391 | 0.254 |

| **Sodium response** | | |
|---|---|---|
| [Na⁺]x10⁻³ M | A₅₀₀ | A₆₀₀ |
| 0.0 | 0.914 | 1.330 |
| 100 | 0.917 | 1.322 |
| 140 | 0.922 | 1.329 |
| 180 | 0.918 | 1.316 |

Under the above conditions the sodium response is insignificant, thus the measurement of lithium can be performed.

### Example 4

A reagent composition was prepared by predissolving 2.5 mg of chromoionophore I in 1 mL of DEGMEE and adding the solution to 100 mL of 1 M KOH containing 1 g of Triton X-100 and 0.23 g of sodium chloride.

2.0 mL of the reagent composition was mixed at room temperature with 0.02 mL of sample and the lithium response was measured with this preferred working reagent composition as in Example 1.

| **Lithium response** | | |
|---|---|---|
| [Li⁺]x10⁻³ M | A₅₀₀ | A₆₀₀ |
| 0.0 | 1.035 | 1.579 |
| 0.5 | 1.099 | 1.433 |
| 1.0 | 1.171 | 1.295 |
| 2.0 | 1.305 | 1.011 |
| 3.0 | 1.442 | 0.721 |
| 4.0 | 1.586 | 0.429 |

Examination of this data shows that the above reagent composition is suitable for the measurement of lithium in serum.

Obviously, many modifications and variations of the invention as herein before set forth can be made without departing from the spirit and scope thereof and therefore only such limitations should be imposed as are indicated by the appended claims.

## Claims

1. A lithium reagent composition comprising: 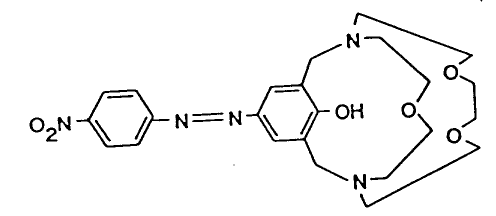 a sodium salt present at a concentration less than 2 x 10⁻² M, a base to maintain the pH at at least 12, a water miscible organic solvent at a concentration of less than 10% volume to volume and a nonionic surfactant at a concentration of less than 10% weight to volume.

2. The composition of claim 1 wherein the sodium salt is sodium chloride.

3. The composition of claim 1 wherein the water miscible organic solvent is diethylene glycol monoethyl ether.

4. A lithium reagent composition comprising: 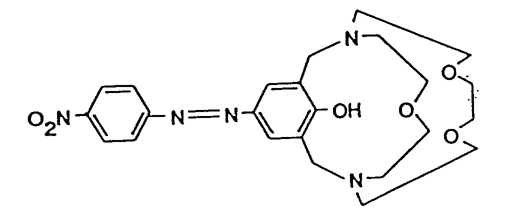 a buffer to control pH to about 11, a water miscible organic solvent at a concentration of less than 10% volume to volume and a nonionic surfactant at a concentration of less than 10% weight to volume.

5. A method for determining lithium ions in a serum test sample comprising the steps of:
(a) contacting said serum test sample with the reagent composition of claim 1;
(b) measuring a detectable colorimetric response; and
(c) comparing the colorimetric response so detected with responses measured when said reagent composition is reacted with a series of standard compositions containing known amounts of lithium ions.

6. A test device for determining the presence and concentration of lithium ions in test sample.
